# EUROPEAN PATENT SPECIFICATION

(11) **EP 1 235 783 B1**
(45) Date of publication and mention of the grant of the patent: **29.09.2004**
(21) Application number: 00957334.6
(22) Date of filing: 08.08.2000
(51) Int. Cl.: C07C 51/41, C07C 51/47, C07C 63/14, C07C 63/337, C07C 63/38

(54) **A PROCESS FOR SEPARATION OF THE DIPOTASSIUM SALT OF NAPHTHALENE DICARBOXYLIC ACID USING ACTIVATED CARBON BEDS**
VERFAHREN ZUR TRENNUNG DES DIKALIUMSALZES DER NAPHTHALENEDICARBONSÄURE MITTELS BETTEN VON AKTIVIERTER KOHLE
PROCEDE DE SEPARATION DU SEL DIPOTASSIQUE DE L'ACIDE DICARBOXYLIQUE DE NAPHTALENE UTILISANT DES LITS DE CHARBON ACTIF

(30) Priority: 30.08.1999 US 151589 P
(43) Date of publication of application: 04.09.2002
(73) Proprietor: Mossi & Ghisolfi International S.A., 1528 Luxembourg (LU)
(72) Inventor: JUNE, Raymond, L., Houston, TX 77079 (US)
(74) Representative: Gerbino, Angelo
(86) International application number: PCT/US2000/021680
(87) International publication number: WO 2001/016079

(56) References cited:
- GB-A- 1 028 852
- GB-A- 1 429 556
- US-A- 3 253 024
- US-A- 5 770 764

## Description

### Technical Field

This invention relates to the purification of 2,6-napthalene dicarboxylic acid. More particularly, this invention relates to a method for separating and recycling unreacted feed from desired product in a process for preparing aromatic dicarboxylic acids of the type which incorporates a disproportionation or rearrangement reaction. In the preferred embodiment, this invention relates to the separation of a salt of naphthoic acid from an aqueous solution containing the disproportionation reaction product comprising the dipotassium salt of 2,6 naphthalene dicarboxylic acid, 2,6 K2NDA, by passing said aqueous solution over activated carbon. It also relates to a method of regenerating the activated carbon beds in place.

### Background Art

Aromatic carboxylic acids are highly useful organic compounds. They are useful as intermediates for the preparation of other organic compounds, and as monomers for the preparation of polymeric materials. In particular, the naphthalene dicarboxylic acids are used for preparing photographic chemicals and dyestuffs. Naphthalene dicarboxylic acids can also be used to prepare a variety of polyester and polyamide compositions. 2,6-naphthalene dicarboxylic acid (hereafter referred to as 2,6-NDA) is a particularly useful aromatic carboxylic acid which can be reacted with ethylene glycol to prepare poly(ethylene-2,6-naphthalate). Polyesters prepared from 2,6-NDA have excellent heat resistance, gas barrier, and mechanical properties. Therefore, much research in the art has focused on methods of preparing 2,6 -NDA. Most of the methods for making 2,6-NDA involve numerous steps, many of which address purification of the product.

It is known in the art to produce the crude dialkali salt of naphthalene-2,6-dicarboxylic acid by heating alkali naphthalene-α-monocarboxylate(alkali 1-naphthoate), alkali naphthalene-β-monocarboxylate(alkali 2-naphthoate), or mixtures thereof at high temperatures in carbon dioxide or other gaseous atmosphere, (German Pat. No. 953,072) or by similarly heating dialkali naphthalene 1,8 dicarboxylate (German Pat. Nos. 932,125 and 1,002,316). This reaction is usually referred to as a Henkel disproportionation reaction and produces a product consisting mainly of a single thermodynamically favored disalt product. It is known in the art to prepare the alkali salts used in this reaction by heating naphthalene α-monocarboxylic acid, naphthalene β-monocarboxylic acid, or naphthalene 1,8-dicarboxylic acid, with hydroxides, carbonates, bicarbonates, etc. of alkali metals in an aqueous medium under heating. When potassium naphthoate is the feed in a Henkel disproportionation reaction, the product is the salt of the desired 2,6-NDA, 2,6-K2NDA. In addition to the desired product, this reaction also typically produces unreacted potassium naphthoate and 2,3 K2NDA. (In describing the invention certain abbreviations will be used which have the following meanings: KNA = potassium naphthoate; 2,6-K2NDA = dipotassium salt of 2,6- naphthalene dicarboxylic acid; and 2,6-NDA = 2,6-naphthalene dicarboxylic acid.)

Methods of separating the desired disalts of 2,6-naphthalene dicarboxylic acid are known. After the Henkel disproportionation reaction, the total reactor effluent is generally dissolved in water to separate out the aromatic core molecule produced by the Henkel disproportionation and the catalyst as separate phases with the unconverted feed and the desired reaction product dissolved in the aqueous phase. In one method known in the art, the disproportionation reaction product, containing the dialkali salt of NDA and the salt of naphthalene mono- or di- carboxylic acid, is dissolved in water, and the water insoluble matters are filtered off. Then a mineral acid is added to the system to release naphthalene carboxylic acids. The free acids are recovered from the system by filtration and are further boiled with an organic solvent such as a lower aliphatic carboxylic acid to separate 2,6-NDA which is insoluble in the organic solvent, from the free dicarboxylic acids which have been separated from the aforementioned unreacted products and side products upon addition of a mineral acid, and are soluble in organic solvent. A disadvantage of this method is that the side product alkali salt of a mineral acid cannot be directly reused in the preparation of starting material for the disproportionation reaction. (See U.S. 2,823,231 and 3,671,578.)

CA 864587 discloses a method for separating 2,6-NDA by disproportionating the alkali salt of 2,6-NDA by heating it in water or water-containing organic solvent to form free 2,6 -NDA and by-product dialkali salt, where the former is precipitated.

One method of purifying 2,6-K2NDA to acceptable levels of potassium naphthoate is by performing two successive crystallizations on the crude 2,6-K2NDA from the disproportionation reaction. This process is energy intensive because the water solubility of 2,6-K2NDA varies only weakly with temperature and the product must be recovered by an evaporative crystallization step which involves evaporating about half of the water charged to the crystallizer. Furthermore, laboratory experiments have shown that the crystallization process cannot recover 2,6-K2NDA in high yield from the crystallization when larger than normal levels of impurities are present in the crystallization feed due to coprecipitation of the impurities with the product 2,6-K2NDA.

None of the references in the art pertaining to production and purification of 2,6-NDA teach the use of activated carbon for separating unreacted feed from product aromatic dicarboxylic acids. U.S. Pat. No. 5,770,764 does disclose the use of carbon beds to remove impurities. In '764 the 2,6-NDA is made by direct oxidation and the carbon bed is used to remove impurities such as trimellitic acid, bromo-2,6-NDA, 2-formyl-6-naphthoic acid, 2 naphthoic acid, other impurities and cobalt and manganese catalyst components.

It is known in the art to use cyclic adsorption to purify petrochemical products. For example, U.S. Pat. No. 3,069,470 discloses the use of type X zeolites for the separation of the meta isomer from other isomers of toluidine.

From U.S. Pat. No. 4,480,129 it is known that X and Y type zeolites, exchanged with transition metals, are paraselective in a mixture of isomers of toluidine.

U.S. Pat. No. 4,642,397 discloses that an adsorbent comprising an X or Y type zeolite cation exchanged with a cation selected from the group K, Na, Ca, Ba, Li, or Mg can be employed to selectively separate 2,4-dinitrotoluene from a feed mixture comprising 2,4-dinitrotoluene and at least one other isomer.

In U.S. Pat. Nos. 5,622,682 and 5,779,998 adsorbents are employed to recover halocarbons from a gas mixture.

Where activated carbon has been used in the art, it is typically used to adsorb trace levels of materials from a solution, rather than percent level impurities in the presence of in the range of ten times excess of a very similar molecule.

Known methods of separating unreacted feed components from 2,6-K2NDA require a number of steps. The prior art for 2,6-NDA purification made by a disproportionation reaction is crystallization or precipitation with carbon dioxide and often the potassium naphthoate is separated in a form which is not recyclable. Therefore, there is a need in the art for a simpler process for separating unreacted feed such as potassium naphthoate from 2,6-K2NDA. This would be a distinct advance in the art relating to purification of 2,6-NDA.

The present invention provides such a process. Other objects and advantages of the invention will be apparent to those skilled in the art from the following detailed description and appended claims.

### Disclosure of the Invention

In accordance with the foregoing, the present invention comprises a process as indicated in claim 1 which follows. Preferred features of the invention are indicated in the dependent claims.

The products to be separated result from a disproportionation or rearrangement type reaction, of the type described in U.S. Pat. Nos. 2,823,231; 3,671,578; and 3,766,258. In the preferred embodiment, the dipotassium salt of 2,6-NDA, 2,6-K2NDA, is separated from unconverted feed components comprising potassium salts of 1- and 2-naphthoic acid in an aqueous solution of the disproportionation reaction product, by passing said aqueous solution over activated carbon, recovering the K2NDA, displacing the adsorbed potassium naphthoate from the activated carbon with a water soluble displacing agent, and regenerating the activated carbon.

### Brief Description Of The Drawings

Figure 1 is a schematic drawing of a potassium naphthoate removal system.
Figure 2 shows as a graph the considerable affinity that CPG activated carbon has for 2-potassium naphthoate.
Figure 3 shows as a graph the amount of adsorption of 2-potassium naphthoate from a simulated disproportionation reactor on one cycle of CPG carbon.
Figure 4 shows as a graph the regeneration of CPG carbon with 20% tetrahydrofuran solution in water.
Figure 5 shows as a graph the sensitivity of the regeneration process to the concentration of tetrahydrofuran.

### Detailed Description Of The Invention

A method known in the art for preparing 2,6-NDA, involves a disproportionation or rearrangement reaction. This reaction disproportionates a feed of aromatic monoacid salts and produces a product comprising mainly a single thermodynamically favored disalt product. Potassium is the economically preferred cation for the salts. A catalyst, typically a Group IIB metal salt or oxide, is used at levels of up to 10 weight percent to increase the disproportionation reaction rate. Additional base, typically as alkali carbonate, can be added to the reaction mixture and helps increase yield of the desired product. After the disproportionation reaction, the product is typically dissolved in water. This separates out the aromatic core molecule produced by the disproportionation reaction, the reaction medium, and the catalyst as separate phases with the unconverted feed and the desired reaction product dissolved in the aqueous phase. As mentioned above, the method for purification of 2,6-NDA has typically been crystallization or precipitation with carbon dioxide, however it has been discovered in the present invention that activated carbon is suitable for use in the separation and recycling of unreacted feed components from salts of aromatic dicarboxylic acids after a disproportionation reaction. After separating the reaction medium(naphthalene) and filtering the catalyst (solid zinc oxide), the dissolved salts are ready for treatment with the solid adsorbent.

In the present invention it has been discovered that in a process for producing 2,6-NDA which incorporates a step of disproportionation or rearrangement of potassium naphthoate to produce the dipotassium salt 2,6-K2NDA, activated carbon can be used as an adsorbent to separate unconverted potassium naphthoate from the desired product.

In addition, it has been discovered in the present invention that the activated carbon can be regenerated using a simple cyclic adsorption process with a facile regeneration accomplished by a wide variety of displacing agents. In the art, spent activated carbon is most often discarded or regenerated via a high temperature thermal process. The temperatures required are typically as high as 900°C and these temperatures would result in the destruction of any organic products adsorbed on the carbon before they could be recycled for conversion into desired products.

Finally, the potassium naphthoate is recovered from the activated carbon beds in a usable form and can be recycled for use in a larger process for making 2,6-NDA.

The results described herein are unexpected because activated carbon is typically used to adsorb trace levels of materials from a solution, not percent level impurities in the presence of about ten times excess of a very similar molecule. It is unexpected for the activated carbon to demonstrate such a strong selectivity difference for two adsorbates that are reasonably similar in molecular weight and structure, such as potassium naphthoate and 2,6-dipotassium naphthalene dicarboxylic acid.

In addition to the alkali metal salt of naphthoic acid, the salts of other aromatic mono- or polycarboxylic acids can be used, however this process is particularly effective for separating unreacted potassium naphthoate from 2,6-dipotassium naphthalene dicarboxylate.

In carrying out the disproportionation of aromatic mono- or dicarboxylic acids it is advantageous to use the alkali metal salts, preferably the potassium salt or the sodium salt is used. The lithium, rubidium and cesium salts can also be used. Cesium is a better cation for the Henkel reaction than potassium, but is very expensive compared to potassium. Potassium works better than sodium and the improved performance supports the price differential between sodium and potassium. Lithium does not work as well as sodium and is extremely toxic. Rubidium is rare in the environment and therefore is very expensive. Also, mixtures of salts of two different metals, such as, sodium and potassium can be used. In the place of the salts, mixtures can also be used which are transformed into the salts upon heating. For example, mixtures of carboxylic acid, anhydrides and alkali metal carbonates can be used. Note however, anhydrides are not likely to run correctly in a Henkel reaction unless the anhydride is mixed with excess alkali and allowed to react to a disalt before heating to Henkel reaction temperatures.

The disproportionation or rearrangement reaction is carried out in an inert, substantially oxygen-free atmosphere in order to prevent decomposition. It is advantageous to perform the rearrangement reaction in an atmosphere of inert gases or vapors such as carbon dioxide, nitrogen, the noble gases, methane, ethane, propane etc., at atmospheric or superatmospheric pressure.

In the preferred embodiment potassium naphthoate is disproportionated in the presence of a thermally stable solvent and a zinc catalyst as described in WO-01/16074.

In the integrated process of WO-01/16074 the aqueous salt is directed to the disproportionation section in a slurry. Just prior to the disproportionation reactor is a two-stage water removal section. To prepare the feed for the disproportionation reaction, water from the aqueous salt solution of potassium naphthoate is evaporated with hot recycled solvent. The water concentration of the disproportionation feed should be reduced to avoid significant decarboxylation of the disproportionation reaction product. The aqueous potassium naphthoate is contacted with solvent to flash water and is then pumped into the disproportionation reactor as a slurry. Just prior to the reactor, the disproportionation reaction Zn catalyst is added to the slurry. The crude product in the slurry with solvent and catalyst are fed into a furnace for heating in preparation for the disproportionation.

Suitable temperatures for the disproportionation reaction are in the range of from about 340°C to 500°. Better results are observed where the temperature is from about 400°C to 480°. The preferred temperature is from about 440°C to 460°C.

Suitable CO₂ pressures for the disproportionation are from about 1,379 to 68,950 kPa (200 to 10,000 psi). A more preferred range is from about 2,413 to 7,584 kPa (350 to 1100 psi). To accelerate the reaction and suppress the occurrence of side reactions the reaction temperature is preferably about 450°C and the pressure is about 5,860 to 6,550 kPa (850 psi to 950 psi).

The disproportionation reaction produces the salt of the desired 2,6-NDA product, (K2NDA), which can include isomers. The disproportionation reaction can include two, or even three, stages to push conversion. The disproportionation effluent solids (in solvent) consist primarily of 2,6-K2NDA, 2,3-K2NDA (isomer intermediate), unreacted KNA, catalyst, and trace coke.

In WO-01/16074 the liquid/solid phase product exits the disproportionation reactor where the pressure is, say about 5,516 to 7,584 kPa (800 to 1100 psi). The liquid/solid phase product exits the disproportionation reactor and enters a tapered bore where there is a significant pressure drop. The result of the pressure letdown is that the solvent is flashed and the liquid/solid phase becomes gas/solid. Then the gas/solid phase products enter the first of two stripper cyclones where each of the two stripper cyclones is connected to a second stage cyclone. Each second stage cyclone is equipped to return additional separated solids to the stripping cyclone to which it is connected. In these cyclones solids are separated and gases exit and are recycled. This is discussed in further detail in U.S. Pat. Appl. Ser. No. 60/151,498 filed August 30, 1999.

The separation of the solvent and catalyst can also be carried out using other methods known in the art, as discussed above.

After separating the solvent and filtering the solid catalyst, the crude product is dissolved in water. At this point the dissolved salts are ready for treating with the solid adsorbent in accordance with the present invention which provides a very energy efficient means of recovering the unreacted potassium naphthoate and separating the dipotassium salts.

Adsorbents that function appropriately for use in the present invention are activated carbons. A large number of commercially available activated carbons are suitable, but preferred results were obtained using those described as high purity carbons. High purity carbons have lowered metal impurities contents, typically less than 2 wt% metals.

Activated carbons employed with good results in Examples 1 through 4 were CPG, 12 X 40 mesh; CAL, 12 x 40 mesh; F400; and PCB, 12 x 30, all commercially available from Calgon Carbon Corporation. Activated carbons can be produced by pyrolyzing organic materials such as coal, peat, or coconut shells under high temperatures in a nonoxidizing environment. The raw carbonaceous material can also be mixed with a binding agent to form a granular material. The pyrolized carbonaceous material can then be activated by steaming to create a high capacity, high surface area adsorbent. Raw materials with low metals content are preferred as they produce more pure activated carbons with a final lower metals content although an acid wash step can be used leach some of the residual metals from the as produced activated carbon.

These carbons are typically washed with acid to remove leachable metals, but still contain percentage levels of metal oxides in the amorphous carbon matrix. In the examples herein, these activated carbons were washed prior to use with dilute potassium hydroxide to prevent the precipitation of insoluble acids on the acidic carbon surfaces by neutralization of the acid sites on the surface of the carbon.

In the process of this invention, the product of a rearrangement reaction, containing unreacted potassium naphthoate and 2,6-dipotassium naphthalene dicarboxylic acid is dissolved in water and passed over activated carbon. Generally, the concentration of the dissolved salts in the aqueous phase is as high as possible to improve the throughput of the process. The concentration of dissolved salts may be from about 10 to 50 wt%, but in the examples of this invention the concentration was about 20-30%, preferably about 25 weight percent 2,6-K2NDA and KNA with the KNA being about 10 wt% on a 2,6-K2NDA basis.

It is desirable to control the pH of the aqueous solution to prevent precipitation of the insoluble carboxylic acids in the activated carbon bed. The pH of the aqueous solution is typically in the range of 10 to 12, but it can be buffered down to as low as about 8.5, if desired, with, for example, carbon dioxide. Lower pH's risk the precipitation of the corresponding carboxylic acids in the activated carbon bed.

The feed is metered to the adsorption beds at a rate such that the mobile phase can equilibrate with the solid phase. For adsorption on carbon from a liquid phase, this is generally about one to three bed weights of aqueous feed per hour. The rate is governed by mass transfer within the carbon particles and from the aqueous solution to the carbon particles. Smaller activated carbon particles increase both mass transfer rates but also dramatically increase the pressure drop across the bed. At equilibrium, bed life is determined by the capacity of the adsorbent for the unconverted feed and the concentration of the unconverted feed in the aqueous stream. The activated carbons investigated herein typically hold 0.25 to 0.30 kg (lb) of 2-KNA per kg (lb) of carbon at equilibrium with a feed solution containing approximately 2 wt% 2-KNA.

The temperature and pressure at which the adsorption columns are run are not critical. The temperature can vary over a wide range, such as, for example, 25°C to 60°C without observing a difference in the capacity of the carbon. Pressure will be determined by factors such as pressure drop requirements for the adsorption beds or by energy conservation strategies within a process unit.

Adsorption runs in the laboratory have shown that, on a 2,6-K2NDA basis, the product can contain as little as 50 ppm 2-KNA after adsorption which is well within the required specification for monofunctional impurities in the final product. The carbon has an added benefit in that it will, to some extent, remove color bodies from the final product by preferential adsorption.

Another embodiment of the present invention comprises regeneration of the carbon beds in place. This embodiment envisions a series of beds of activated carbon, such that the aqueous solution can be contacted with a first bed until the adsorbent in the bed is expended. Then the bed is taken off-line and the aqueous solution of disproportionation reaction product is fed to another adsorption bed in the adsorption train.

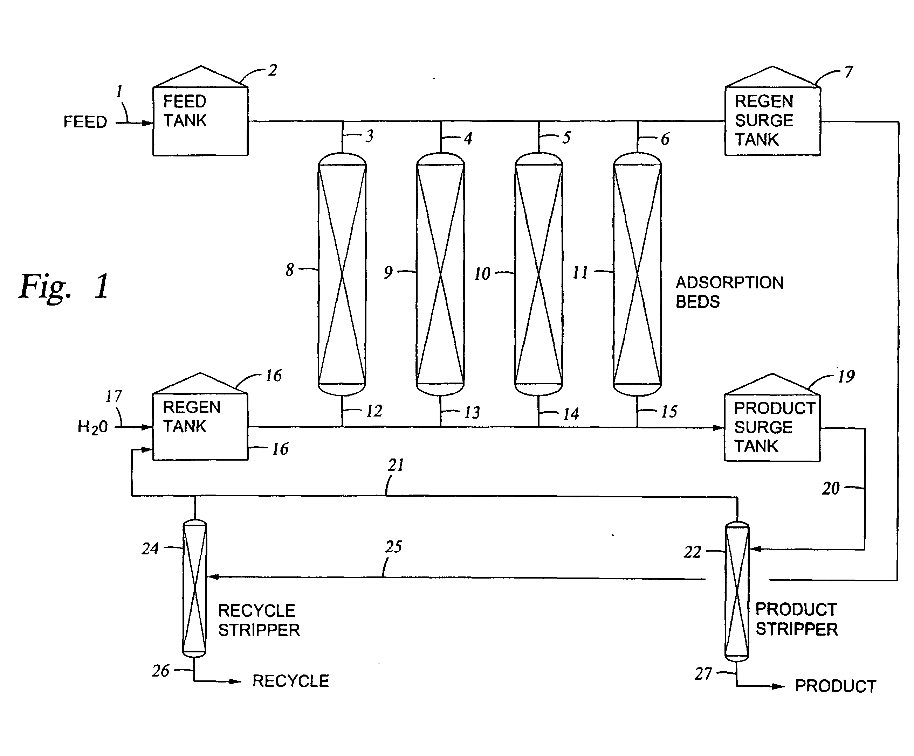

The regeneration of the carbon beds in place is a very attractive feature of this invention. Referring to Figure 1, it is noted that a number of beds can be operated together in a carousel fashion to achieve pseudo steady state behavior on the product and regeneration streams. When a bed is taken off-line, the spent bed is preferably backwashed first with about 1 bed volume of water to displace any 2,6-K2NDA or other unadsorbed feed material back to the feed tank for the adsorption unit. This step helps to increase the per pass yield of the adsorption section by minimizing the amount of 2,6-K2NDA that is removed from the adsorption bed in the regeneration stream. Additionally, it is also preferred to operate the backwash and regeneration steps in a countercurrent fashion to the adsorption step to maximize the efficiency of the adsorption bed.

Regeneration in laboratory column experiments has been performed with a solution of displacing agent in water in countercurrent flow at a rate of 1-3 bed weights per hour. The displacing agent is an organic molecule that has a reasonable affinity for adsorption on activated carbon and is capable of displacing the weakly bound potassium naphthoate from the carbon.

The displacing agent can suitably be selected from water soluble alcohols, esters or acids having one to four carbon atoms. Examples include, but are not limited to, methanol, acetone, isopropyl alcohol, tetrahydrofuran, and ethyl acetate. P-xylene works well as a displacing agent, but is not water soluble. While many organics can displace the KNA's from the activated carbon, a significant amount of water is required to solubilize the desorbed KNA's. A mass balance on the effluent of the adsorption and desorption experiments indicates that this achieves essentially complete regeneration of the bed.

Good results were achieved regenerating the activated carbon beds using tetrahydrofuran. Using 20% tetrahydrofuran in water in the regeneration stream, a bed of adsorbent can be regenerated with about 15 bed weights of regenerant solution. The tetrahydrofuran can be easily removed from the aqueous solution of the desorbed potassium naphthoate by steam stripping and can be recycled for desorption. After the regeneration step is complete, it is likely that a second backwash step of about 1 bed weight of water should be performed. After regeneration, the activated carbon can be reused to treat more disproportionation reaction product.

A drawing of a process incorporating the adsorption methods of this invention is shown in Fig. 1. An aqueous solution of KNA and K2NDA 1 from which solvent and catalyst has been removed enters the feed tank 2. Optionally, the feed to tank 2 can be pH adjusted by the addition of acid of base to the solution to maintain the tank contents in a desired pH region. The aqueous solution then enters through line **3** to the first of a series, **8**, of adsorption beds, **8-11.** The K2NDA exits through lines **12-15** and enters a product surge tank, **19.** The product is then directed through line 20 to a product stripper **22**, where the regeneration solvent is removed in an overhead stream **21** and the K2NDA product exits as a bottoms product free, or substantially free, of regeneration solvent at **27**. The number of four beds is just used for illustration; the invention contemplates more or less. The adsorption beds are manifolded with valves in such a way that feed can be put into any one of the beds and product from that bed can be fed to any other bed in the train. Furthermore, the manifolding allows for the isolation of a bed during the regeneration phase. The piping manifold is designed so as to minimize dead volume and retained deadlegs which can substantially reduce the efficiency of the adsorption beds. When the activated carbon in the first adsorption bed **8** is depleted, feed coming in is then directed through line **4** into bed **9;** then through line **5** to bed **10,** and line **6** to bed **11,** etc. Another aspect of the invention is that the adsorption beds are regenerated in place. Water is added as needed by **17** to makeup tetrahydrofuran in the regeneration tank **16** and directed countercurrently through line **12** into bed **8** after it has been taken off line and the displacing agent exits **3** with KNA and is directed to the regeneration surge tank **7**. The solution of displacing agent and KNA is then directed from **7** to the recycle stripper, **24** where the KNA solution is stripped with steam or other inert gas (nitrogen, methane, etc.) and can be recycled in an integrated NDA process via **26**, and the displacing agent is returned to the regeneration tank via **21**. The water containing overheaded regeneration solvent is directed to tank 16 for reuse in the separation process.

The following examples will serve to illustrate specific embodiments of the invention disclosed herein. These examples are intended only as a means of illustration and should not be construed as limiting the scope of the invention in any way. Those skilled in the art will recognize many variations which may be made without departing from the spirit of the disclosed invention.

### EXAMPLES

A number of laboratory scale experiments were performed in both batch contacting mode to screen adsorbents and in a flow column mode to investigate the behavior of the adsorbent/solution system. Using the batch data adsorbents can be quickly screened. Data are also useful to generate adsorption isotherms which can be used to at least qualitatively predict system behavior in an adsorption column.

### EXAMPLES 1-12

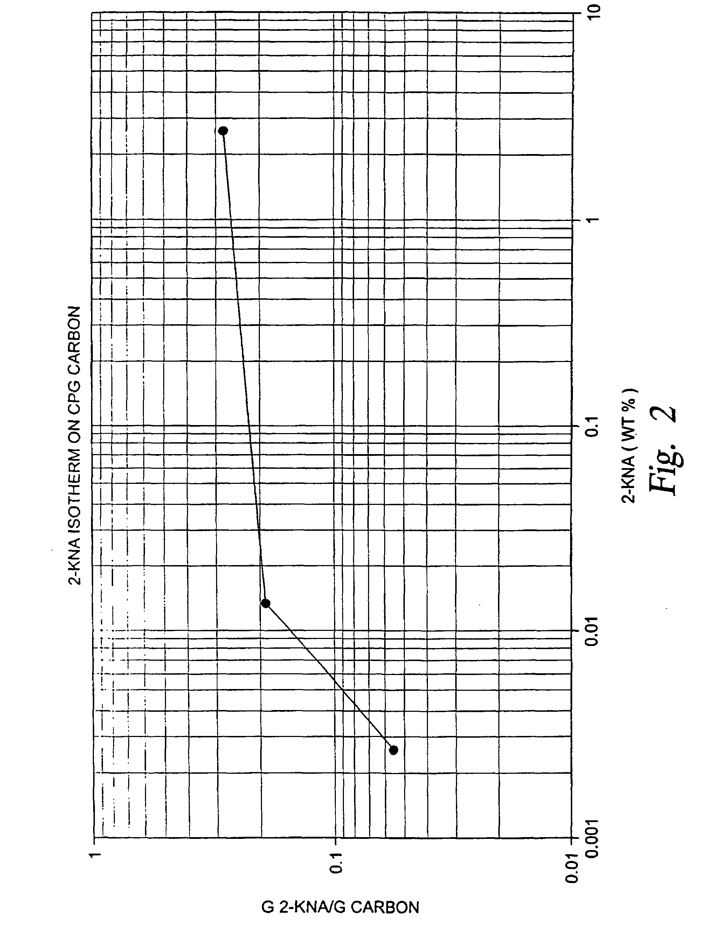

Batch screening experiments were performed by stirring solutions of 2-KNA or 2,6-K2NDA or combinations of solutes both in glass vials with an adsorbent sample overnight at 25 °C in a phase ratio of 10 to 1. Typically, the impurities (1-KNA, 2-KNA, or 2,3-K2NDA) were charged at 2% in the aqueous solutions while 2,6-K2NDA was charged at 15-20% to determine the selectivity of the adsorbents for the impurities species in the presence of large amounts of 2,6-K2NDA. Before testing, the adsorbents were prepared by washing with deionized water and oven drying to get an accurate weight on the adsorbent phase. These batch screening experiments show that the activated carbons possess the best adsorption capacity. An adsorption isotherm for the adsorption of 2-KNA on CPG carbon is presented in Figure 2 and demonstrates graphically the considerable affinity that CPG carbon has for 2-KNA.The polymeric adsorbent resins have some capacity for 2-KNA, but it is not as good as the activated carbons. The zeolitic adsorbents possessed poor capacity for 2-KNA and are expected to perform poorly. Furthermore, the zeolites are somewhat unstable in basic solutions as they tend to slowly dissolve.

A large number of activated carbons are commercially available, but the focus was on "high purity" carbons to minimize metals contamination of the adsorption product. These carbons are typically washed with acid to remove leachable metals, but still contain percent levels of metal oxides in the amorphous carbon matrix. To prevent the precipitation of the insoluble acids on the carbons by neutralization with acid sites on the surface of the carbon, a procedure was employed wherein the carbons were first washed with dilute potassium hydroxide. The caustic wash can be carried out in batch or continuous mode and involves letting the activated carbon sit in the presence of a dilute potassium hydroxide solution before rinsing with deionized water to remove entrained potassium hydroxide.

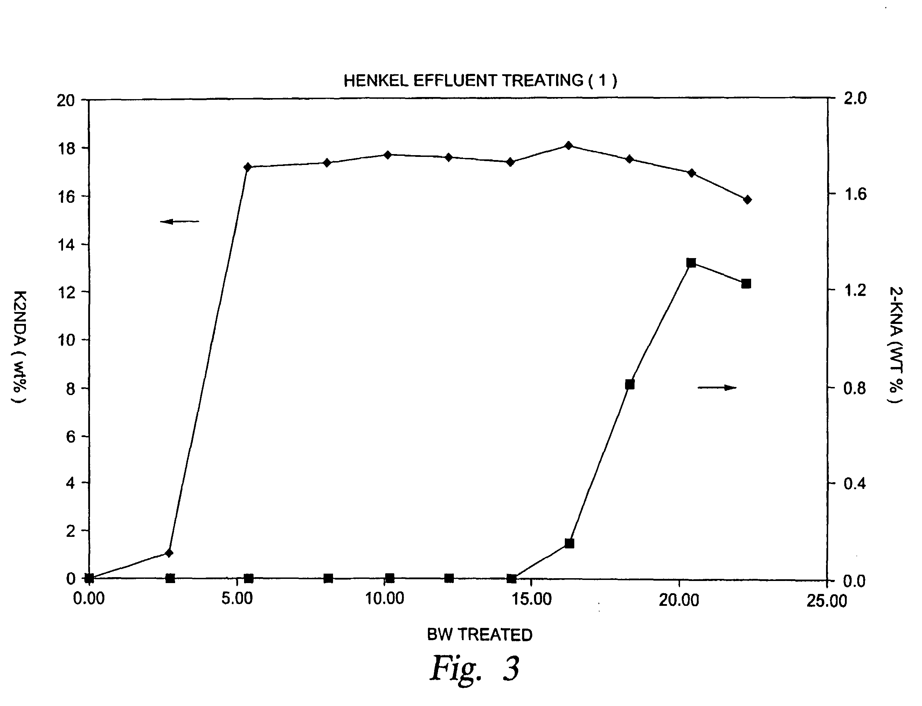

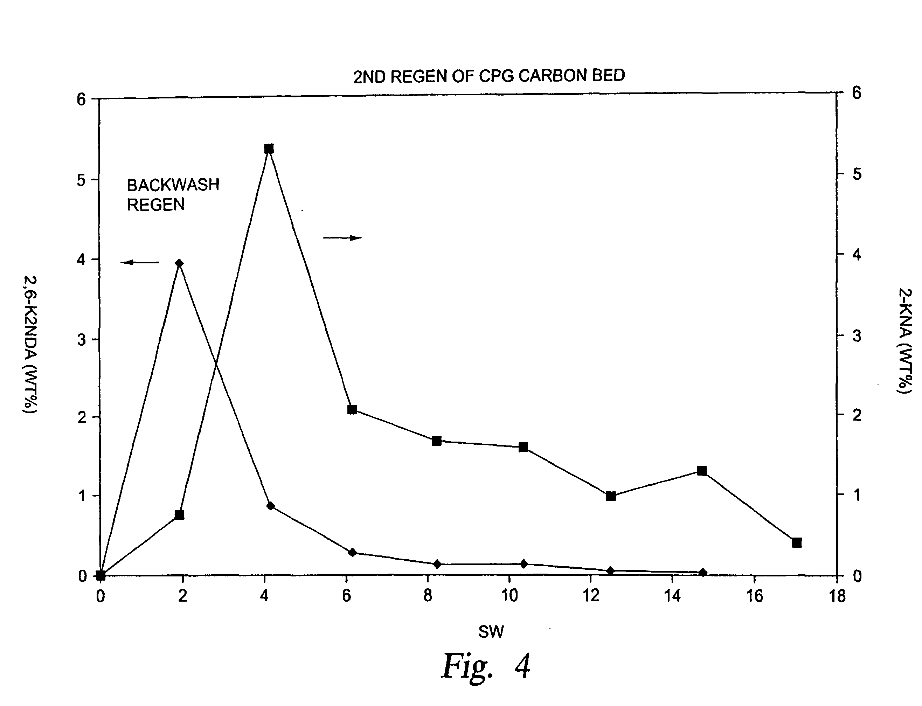

The flow adsorption experiments consisted of passing an aqueous solution of 2-KNA and 2,6-K2NDA through a 5/8 inch diameter glass jacketed column containing activated carbon. The bed temperature was maintained at 40°C by circulating warm ethylene glycol through the column jacket. The feed solution was pumped through the column at weight hourly space velocities (WHSV's) of 1-4. After charging a dried and weighed activated carbon sample to the column, the bed was degassed by flooding the bed under vacuum to removed trapped air. During operation, the effluent was routinely sampled to determine the levels of impurities in the effluent via ion chromatography. After the treating operation had the bed was loaded with monofunctional impurities, the regeneration step was tested by feeding a 20% tetrahydrofuran in water solution. Regeneration was performed counter current to the adsorption operation. Figs. 3 and 4 show the concentrations of 2,6-K2NDA and 2-KNA in the outlet of the column under adsorption and regeneration conditions, respectively. Ion chromatography of effluent samples determined that 2-KNA levels were as low as 50 ppm (on K2NDA) in the adsorption effluent. By operating the bed in a counter current mode, both good recovery and purity are obtained in the adsorption process.

Table 1 gives a list of the adsorbents tested in the laboratory and the adsorbates tested with those adsorbents. Although both 1- and 2-KNA are expected to be in the disproportionation reactor effluent when mixed methyl-naphthalenes are used as naphthoic acid precursor, 2-KNA was primarily used in the examples for consistency in analyzing the results.

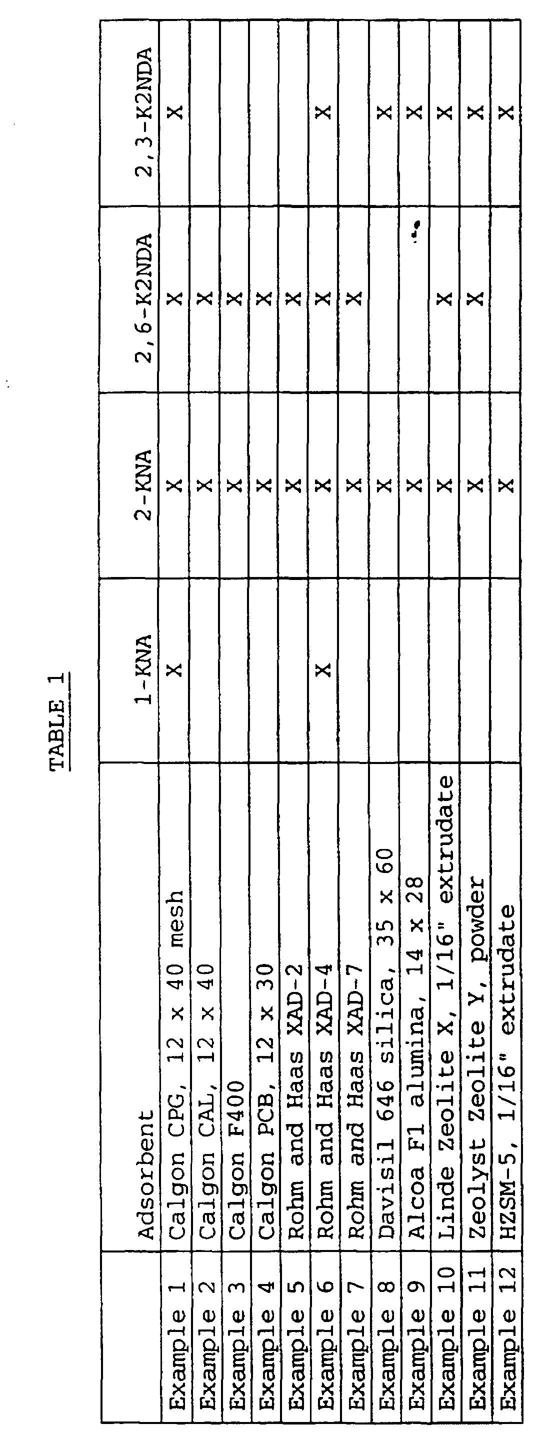

### EXAMPLES 13-19

In Examples 13 through 19 different displacing agents were tested. In these examples, a 5% solution of 2-KNA dissolved in water was contacted with a CPG carbon in a 10:1 ratio. The two phases were contacted overnight. After the equilibration period, 20% by weight of the displacing agent was added to the solution and stirred for 4 hours. A sample was then taken to determine which of the displacing agents was the most efficient. The results are recorded in Table 2.

**TABLE 2**

| | Displacing Agent | Wt% 2-KNA |
|---|---|---|
| Example 13 | P-xylene | 4.57 |
| Example 14 | Methanol | 2.70 |
| Example 15 | Acetone | 3.38 |
| Example 16 | Isopropyl Alcohol | 3.42 |
| Example 17 | Tetrahydrofuran | 3.96 |
| Example 18 | Ethylacetate | 3.48 |
| Example 19 | Blank Control | 2.52 |

As can be seen from the data in Table 2, p-xylene was the strongest displacing agent, but it is not water soluble. It is assumed that for reliable operation in an adsorption column that a single phase would be required. Of the water soluble organics, tetrahydrofuran was the most efficient at displacing 2-KNA from the activated carbon. As a cyclic ether, tetrahydrofuran also has the advantage that it is reasonably stable against hydrolysis or esterification.

### EXAMPLE 20

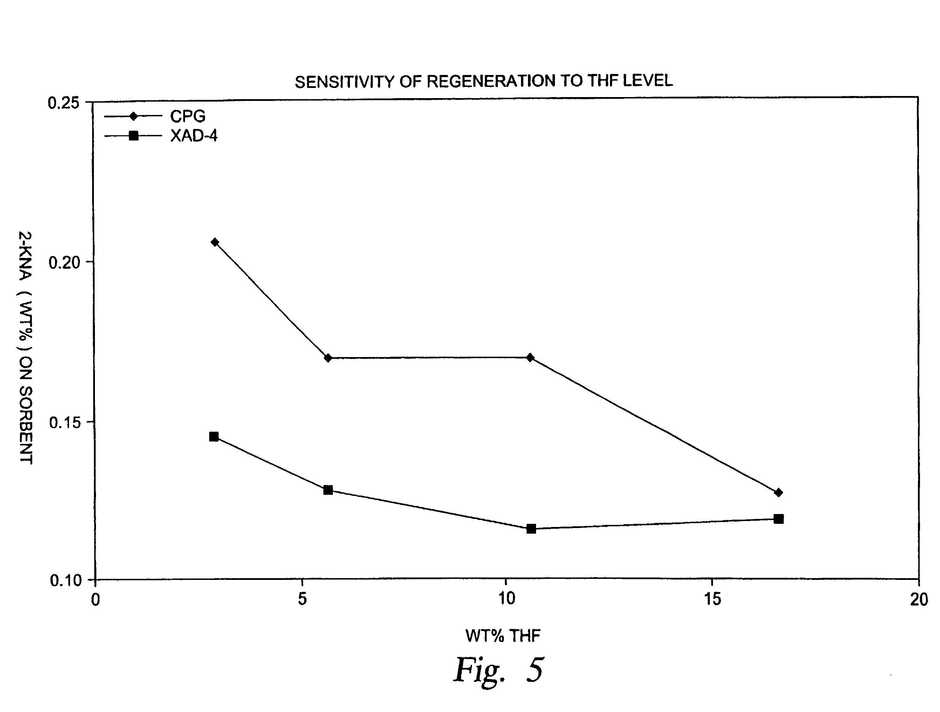

In this example, an activated carbon and adsorbent resin, Rohm and Haas XAD-4, were equilibrated with 2-KNA solutions. Then varying levels of tetrahydrofuran were added to the supernatant solution to displace the 2-KNA from the adsorbent. The results are shown in the graph in Figure 5. The graph in Figure 5 shows that reasonably high levels of displacing agent are required to remove 2-KNA from the solid adsorbents. Even at levels of tetrahydrofuran approaching 20 wt%, the carbon still contains as much as 10 wt% 2-KNA. This demonstrates that the 2-KNA is more strongly sorbed and that the displacing agent works largely by mass action.

## Claims

1. Process for producing aromatic dicarboxylic acids which incorporates disproportionation of a salt of an aromatic carboxylic acid to produce unreacted salt of an aromatic carboxylic acid and the salt of the desired aromatic dicarboxylic acid, said process comprising the steps of separating the salt of the desired product from the unreacted salt which is present in percent level by passing both said unreacted salt and said salt of the desired product in an aqueous solution over a bed of adsorbent comprising activated carbon, regenerating the expended bed and recycling the separated unreacted salt.

2. The process of Claim 1 wherein the unreacted salt is potassium naphthoate, and the salt of the desired product is the dipotassium salt of naphthalene dicarboxylic acid 2,6-K2NDA.

3. The process of Claim 1 wherein the aqueous solution of unreacted potassium naphthoate and dipotassium salt of 2,6-naphthalene dicarboxylic acid contains about 1-50% of the combined salts in water.

4. The process of Claim 3 wherein the aqueous solution contains about 10-40% combined salts in water.

5. The process of Claim 4 wherein the aqueous solution contains about 20-30% combined salts in water.

6. The process of Claim 1 wherein said adsorbent is activated carbon.

7. The process of Claim 6 wherein said activated carbon adsorbent further comprises a high purity carbon that has been washed to remove leachable metals.

8. The process of Claim 7 wherein said high purity activated carbon is washed with an aqueous potassium hydroxide solution before first use.

9. The process of Claim 1 wherein said adsorbent bed further comprises multiple adsorbent beds in a series.

10. The process of Claim 9 wherein said aqueous solution is treated over an adsorbent bed until said bed is expended and then said aqueous solution is directed to the next adsorbent bed in the series.

11. The process of Claim 10 which further comprises regenerating the expended bed by countercurrently backwashing the bed with water and then countercurrently passing displacing agent over the bed.

12. The process of Claim 11 wherein the bed is countercurrently backwashed with about 1-3 bed volumes of water.

13. The process of Claim 11 further comprising passing said displacing agent countercurrently over the bed at a rate of about 0.1-10 bed weights per hour.

14. The process of Claim 11 wherein said displacing agent selected from the group consisting of at least partially water soluble alcohols, ethers or esters **characterized by** having from one to six carbons.

15. The process of Claim 14 wherein the water soluble displacing agent is selected from the group consisting of methanol, acetone, isopropyl alcohol, ethyl acetate, and tetrahydrofuran.

16. The process of Claim 15 wherein the water soluble displacing agent is tetrahydrofuran.

17. The process of Claim 11 wherein said displacing agent is in a solution of water in an amount of 5-80%.

18. The process of Claim 17 wherein said displacing agent is in a solution of water in an amount of 15-50%.

19. The process of Claim 18 wherein said displacing agent is in a solution of water in an amount of about 20% or the solubility limit of the solvent in the aqueous solution, whichever is higher.

20. Process according to claim 1 for producing 2, 6-naphthalene dicarboxylic acid by disproportionation of potassium naphthoate to produce the dipotassium salt of 2,6 -naphthalene dicarboxylic acid and unreacted 1- and 2- potassium napthoate, which process provides for separating said potassium naphthoate from the dipotassium salt of napthalene dicarboxylic acid by:
passing both said potassium naphthoate and said dipotassium salt in an aqueous solution over the first of a series of two to six beds containing adsorbent comprising activated carbon until said activated carbon is expended;
directing said aqueous solution to the second bed, or subsequent bed in the series, of activated carbon;
backwashing the expended bed with water; and
regenerating the expended bed by countercurrently passing a saturated solution of tetrahydrofuran in water over the expended bed.

## Patentansprüche

1. Verfahren zur Herstellung aromatischer Dicarbonsäuren, welches das Disproportionieren eines Salzes einer aromatischen Carbonsäure zwecks Herstellung eines nicht umgesetzten Salzes einer aromatischen Carbonsäure und des Salzes der gewünschten aromatischen Dicarbonsäure einschließt, wobei das Verfahren die Schritte des Abtrennens des Salzes des gewünschten Produkts vom in Prozenthöhe vorhandenen, nicht umgesetzten Salz umfasst, indem sowohl das nicht umgesetzte Salz als auch das Salz des gewünschten Produkts in einer wässrigen Lösung über ein Aktivkohle umfassendes Adsorptionsmittelbett geleitet werden, sowie des Regenerierens des verbrauchten Betts und des Zurückführens des abgetrennten, nicht umgesetzten Salzes.

2. Verfahren nach Anspruch 1, wobei das nicht umgesetzte Salz Kaliumnaphthoat und das Salz des gewünschten Produkts das Dikaliumsalz von Naphthalindicarbonsäure, 2,6-K2NDA, ist.

3. Verfahren nach Anspruch 1, wobei die wässrige Lösung von nicht umgesetztem Kaliumnaphthoat und Dikaliumsalz von 2,6-Naphthalindicarbonsäure etwa 1-50% kombinierte Salze in Wasser enthält.

4. Verfahren nach Anspruch 3, wobei die wässrige Lösung etwa 10-40% kombinierte Salze in Wasser enthält.

5. Verfahren nach Anspruch 4, wobei die wässrige Lösung etwa 20-30% kombinierte Salze in Wasser enthält.

6. Verfahren nach Anspruch 1, wobei das Adsorptionsmittel Aktivkohle ist.

7. Verfahren nach Anspruch 6, wobei das Aktivkohle-Adsorptionsmittel weiters einen hochreinen Kohlenstoff umfasst, welcher gewaschen wurde, um auslaugbare Metalle zu entfernen.

8. Verfahren nach Anspruch 7, wobei die hochreine Aktivkohle vor der ersten Verwendung mit einer wässrigen Kaliumhydroxidlösung gewaschen wird.

9. Verfahren nach Anspruch 1, wobei das Adsorptionsmittelbett weiters mehrere Adsorptionsmittelbetten in Serie umfasst.

10. Verfahren nach Anspruch 9, wobei die wässrige Lösung über einem Adsorptionsmittelbett behandelt wird, bis das Bett verbraucht ist, und die wässrige Lösung danach zum nächsten Adsorptionsmittelbett in Serie geleitet wird.

11. Verfahren nach Anspruch 10, welches weiters das Regenerieren des verbrauchten Betts umfasst, indem das Bett im Gegenstrom mit Wasser rückgewaschen wird und danach Verdrängungsmittel im Gegenstrom über das Bett geleitet wird.

12. Verfahren nach Anspruch 11, wobei das Bett im Gegenstrom mit etwa 1-3 Bettvolumen Wasser rückgewaschen wird.

13. Verfahren nach Anspruch 11, weiters umfassend das Leiten des Verdrängungsmittels im Gegenstrom über das Bett, und zwar in einer Geschwindigkeit von etwa 0,1-10 Bettgewichten pro Stunde.

14. Verfahren nach Anspruch 11, wobei das Verdrängungsmittel ausgewählt ist aus der Gruppe, bestehend aus zumindest teilweise wasserlöslichen Alkoholen, Ethern oder Estern, die **dadurch gekennzeichnet sind, dass** sie ein bis sechs Kohlenstoffe aufweisen.

15. Verfahren nach Anspruch 14, wobei das wasserlösliche Verdrängungsmittel ausgewählt ist aus der Gruppe, bestehend aus Methanol, Aceton, Isopropylalkohol, Ethylacetat und Tetrahydrofuran.

16. Verfahren nach Anspruch 15, wobei das wasserlösliche Verdrängungsmittel Tetrahydrofuran ist.

17. Verfahren nach Anspruch 11, wobei das Verdrängungsmittel in einer Menge von 5-80% in einer Wasserlösung ist.

18. Verfahren nach Anspruch 17, wobei das Verdrängungsmittel in einer Menge von 15-50% in einer Wasserlösung ist.

19. Verfahren nach Anspruch 18, wobei das Verdrängungsmittel in einer Menge von etwa 20% in einer Wasserlösung oder entsprechend der Löslichkeitsgrenze des Lösungsmittels in der wässrigen Lösung ist, je nachdem, was höher ist.

20. Verfahren gemäß Anspruch 1 zur Herstellung von 2,6-Naphthalindicarbonsäure durch Disproportionierung von Kaliumnaphthoat zwecks Herstellung des Dikaliumsalzes von 2,6-Naphthalindicarbonsäure und von nicht umgesetztem 1- und 2-Kaliumnaphthoat, welches Verfahren für eine Abtrennung des Kaliumnaphthoats vom Dikaliumsalz der Naphthalindicarbonsäure sorgt, indem:
sowohl das Kaliumnaphthoat als auch das Dikaliumsalz in einer wässrigen Lösung über das erste in einer Serie von zwei bis sechs Betten, welche ein Aktivkohle umfassendes Adsorptionsmittel enthalten, geleitet werden, bis die Aktivkohle verbraucht ist;
die wässrige Lösung zum zweiten Bett oder zum darauffolgenden Bett aus Aktivkohle in der Serie geleitet wird;
das verbrauchte Bett mit Wasser rückgewaschen wird; und
das verbrauchte Bett regeneriert wird, indem eine gesättigte Tetrahydrofuranlösung in Wasser im Gegenstrom über das verbrauchte Bett geleitet wird.

## Revendications

1. Procédé de production d'acides dicarboxyliques aromatiques qui intègre la dismutation d'un sel d'un acide carboxylique aromatique pour produire du sel ayant non réagi d'un acide carboxylique aromatique et le sel de l'acide dicarboxylique aromatique désiré, ledit procédé comprenant les étapes consistant à séparer le sel du produit désiré du sel ayant non réagi qui est présent en un niveau de pourcentage en faisant passer à la fois ledit sel ayant non réagi et ledit sel du produit souhaité dans une solution aqueuse par-dessus un lit d'adsorbant comprenant du charbon actif, à régénérer le lit expansé et à recycler le sel ayant non réagi séparé.

2. Procédé selon la revendication 1, dans lequel le sel ayant non réagi est du naphthoate de potassium, et le sel du produit désiré est le sel dipotassique de l'acide naphthalène dicarboxylique 2,6-K2NDA.

3. Procédé selon la revendication 1, dans lequel la solution aqueuse du naphthoate de potassium ayant non réagi et du sel dipotassique de l'acide 2,6-naphthalène dicarboxylique contient environ 1 à 50 % des sels combinés dans de l'eau.

4. Procédé selon la revendication 3, dans lequel la solution aqueuse contient environ 10 à 40 % des sels combinés dans de l'eau.

5. Procédé selon la revendication 4, dans lequel la solution aqueuse contient environ 20 à 30 % des sels combinés dans de l'eau.

6. Procédé selon la revendication 1, dans lequel ledit adsorbant est du charbon actif.

7. Procédé selon la revendication 6, dans lequel ledit adsorbant à base de charbon actif comprend en outre un charbon de pureté élevée qui a été lavé pour éliminer les métaux lixiviables.

8. Procédé selon la revendication 7, dans lequel ledit charbon actif de pureté élevée est lavé avec une solution aqueuse d'hydroxyde de potassium avant le premier emploi.

9. Procédé selon la revendication 1, dans lequel ledit adsorbant comprend en outre des lits adsorbants multiples en série.

10. Procédé selon la revendication 9, dans lequel ladite solution aqueuse est traitée sur un lit adsorbant jusqu'à ce que ledit lit soit expansé et ensuite ladite solution aqueuse est dirigée vers le lit adsorbant suivant dans la série.

11. Procédé selon la revendication 10, qui comprend en outre la régénération du lit expansé par un lavage à contre-courant du lit avec de l'eau, et ensuite à faire passer à contre-courant un agent de déplacement par-dessus le lit.

12. Procédé selon la revendication 11, dans lequel le lit est lavé à contre-courant avec environ 1 à 3 fois le volume du lit en eau.

13. Procédé selon la revendication 11, comprenant en outre le passage dudit agent de déplacement à contre-courant par-dessus le lit à une vitesse d'environ 0,1 à 10 fois le poids du lit par heure.

14. Procédé selon la revendication 11, dans lequel ledit agent de déplacement est choisi au sein du groupe constitué d'alcools, d'éthers ou d'esters au moins partiellement solubles dans l'eau, **caractérisés par le fait qu'**ils ont de un à six atome(s) de carbone.

15. Procédé selon la revendication 14, dans lequel l'agent de déplacement soluble dans l'eau est choisi au sein du groupe constitué du méthanol, de l'acétone, de l'alcool isopropylique, de l'acétate d'éthyle, et du tétrahydrofuranne.

16. Procédé selon la revendication 15, dans lequel l'agent de déplacement soluble dans l'eau est le tétrahydrofuranne.

17. Procédé selon la revendication 11, dans lequel ledit agent de déplacement est en solution dans l'eau en une quantité de 5 à 80 %.

18. Procédé selon la revendication 17, dans lequel ledit agent de déplacement est en solution dans l'eau en une quantité de 15 à 50 %.

19. Procédé selon la revendication 18, dans lequel ledit agent de déplacement est en solution dans l'eau en une quantité d'environ 20 % ou à la limite de solubilité du solvant dans la solution aqueuse, aussi élevée qu'elle soit.

20. Procédé selon la revendication 1, pour produire de l'acide 2,6-naphthalène dicarboxylique par dismutation de naphthoate de potassium pour produire le sel dipotassique de l'acide 2,6-naphthalène dicarboxylique et du 1- et 2-naphthoate de potassium ayant non réagi, lequel procédé propose la séparation dudit naphthoate de potassium du sel dipotassique de l'acide naphthalène dicarboxylique à travers les étapes consistant à :
faire passer à la fois ledit naphthoate de potassium et ledit sel dipotassique dans une solution aqueuse par-dessus le premier d'une série de deux à six lits contenant l'adsorbant comprenant le charbon actif jusqu'à ce que ledit charbon actif soit expansé ;
diriger ladite solution aqueuse vers le second lit, ou le lit ultérieur dans la série, de charbon actif ;
laver à contre-courant le lit expansé avec de l'eau ; et
régénérer le lit expansé en faisant passer à contre-courant une solution saturée de tétrahydrofuranne dans de l'eau par-dessus le lit expansé.
